# EUROPEAN PATENT APPLICATION

(11) **EP 2 402 010 A1**
(43) Date of publication of application: **04.01.2012**
(21) Application number: 11007870.6
(22) Date of filing: 11.12.2003
(51) Int. Cl.: A61K 31/4045, A61K 45/00, A61K 47/10, A61K 47/12, A61K 9/20, A61K 9/28, A61K 9/48, A61P 13/00, A61P 13/02, A61P 43/00, A61K 9/16

(54) **Solid drug for oral use**

(30) Priority: 16.12.2002 JP 2002364238
(62) Divisional of application: 03780719.5
(71) Applicant: Kissei Pharmaceutical Co., Ltd., Matsumoto-City Nagano-Pref. 399-8710 (JP)
(72) Inventor: Naganuma, Tsuyoshi, Minamiazumi-gun Nagano 399-8205 (JP); Muramatsu, Mitsuo, Tokyo 173-0001 (JP)
(74) Representative: Hayes, Adrian Chetwynd

(57) **Abstract**

The present invention provides a tablet for the treatment of dysuria, which is coated with a light-shielding coating agent containing titanium oxide and comprises a granule containing a) as an active ingredient, an indoline compound having an α₁-adrenoceptor blocking activity and represented by the formula: 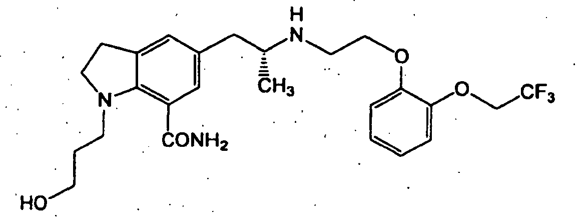 b) D-mannitol as a filler, c) low substituted hydroxypropylcellulose as a disintegrator, and d) hydroxypropylcellulose as a binder,
wherein 85% dissolution time is not more than 15 minutes in a dissolution test according to method 2 (paddle method) of the Japanese pharmacopoeia in a condition using water as a test medium and a paddle speed of 50rpm.

## Description

### TECHNICAL FIELD

The present invention relates to a solid oral dosage form pharmaceutical for the treatment of dysuria. More particularly, the present invention relates to a solid oral dosage form pharmaceutical for the treatment of dysuria, which comprises, as an active ingredient, an indoline compound having an α₁ -adrenoceptor (hereinafter referred to as "α₁-AR") blocking activity and represented by the formula (I) (hereinafter referred to as "KMD-3213"): 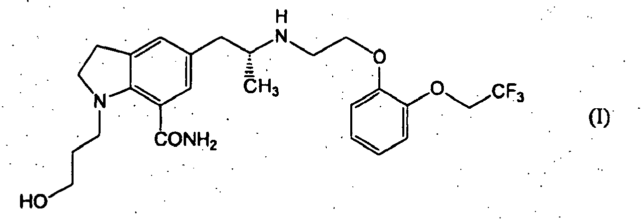 its prodrug, pharmaceutically acceptable salt or pharmaceutically acceptable solvate thereof, wherein 85% dissolution time is not more than 60 minutes in a dissolution test according to method 2 (paddle method) of Japanese pharmacopoeia in a condition using water as a test medium and a paddle speed of 50rpm.

The present invention also relates to a solid oral dosage form pharmaceutical for the treatment of dysuria, said pharmaceutical comprising, as an active ingredient, 1) KMD-3213, its prodrug, pharmaceutically acceptable salt or pharmaceutically acceptable solvate thereof, and 2) at least one selected from the group consisting of an α₁-adrenoceptor blocking agent, an anticholinergic agent, an antiinflammatory agent and an antibacterial agent other than KMD-3213, wherein 85% dissolution time is not more than 60 minutes in a dissolution test according to method 2 (paddle method) of Japanese pharmacopoeia in a condition using water as a test medium and a paddle speed of 50rpm.

The present invention also relates to a solid oral dosage form pharmaceutical and a kit which comprises:
1) a pharmaceutical for the treatment of dysuria comprising, as an active ingredient, KMD-3213, its prodrug, pharmaceutically acceptable salt or pharmaceutically acceptable solvate thereof, wherein 85% dissolution time is not more than 60 minutes in a dissolution test according to method 2 (paddle method) of Japanese pharmacopoeia in a condition using water as a test medium and a paddle speed of 50rpm, in combination with
2) a pharmaceutical comprising, as an active ingredient, at least one selected from the group consisting of an α₁-adrenoceptor blocking agent, an anticholinergic agent, an antiinflammatory agent and an antibacterial agent other than KMD-3213.

When solid oral dosage form pharmaceuticals for the treatment of dysuria of the present invention are tested for their dissolution properties according to a dissolution test, method 2 (paddle method) of Japanese pharmacopoeia in a condition using water as a test medium and a paddle speed of 50rpm, 85% dissolution time (hereinafter referred to as "T85%") of said pharmaceuticals is preferably not more than 60 minutes. More preferably, T85% of the present pharmaceuticals is not more than 60 minutes when tested according to method 2 (paddle method) of Japanese pharmacopoeia in a condition using the first fluid regulated in a disintegration test of Japanese pharmacopoeia (hereinafter referred to as "the first fluid") as a test medium and a paddle speed of 50rpm. Even more preferably, T85% of the present pharmaceutical is not more than 30 minutes, and most preferably is not more than 15 minutes when tested according to method 2 (paddle method) of Japanese pharmacopoeia in a condition using water or the first fluid.

The first fluid employed in a dissolution test of the present invention refers to the first fluid regulated in a disintegration test of Japanese pharmacopoeia, wherein the first fluid is prepared by adding 2.0g of sodium chloride to 7.0mL of hydrochloric acid and water to make a 1000mL of test medium.

### BACKGROUND ART

It is known that KMD-3213, which is contained as an active ingredient in a solid oral dosage form pharmaceutical for the treatment of dysuria of the present invention, has selective suppressing activities on the contraction of urethra smooth muscles, and is an extremely useful compound as a medicament for treating dysuria without causing strong hypotensive activities or orthostatic hypotension.

As for pharmaceutical compositions comprising, as an active ingredient, KMD-3213, pharmaceutically acceptable salt or pharmaceutically acceptable solvate thereof, the following literatures have been known so far.

In patent literature 1, which discloses indoline compounds including KMD-3213, several dosage forms are exemplified as an oral solid formulation. It is also reported therein as a general description that such dosage forms may be prepared by formulating indoline compounds according to conventional formulation procedures. However, patent literature 1 has not disclosed a specific formulation comprising, as an active ingredient, KMD-3213.

In patent literature 2, which discloses a medicament comprising, as an active ingredient, an α₁-AR blocking agent including KMD-3213 for treating lower urinary tract disorders, several dosage forms are exemplified as an oral solid formulation. It is also reported that such dosage forms may be prepared using ordinary pharmaceutical additives according to conventional formulation procedures. However, patent literature 2 has not disclosed a specific pharmaceutical composition comprising, as an active ingredient, KMD-3213.

KMD-3213 is relatively unstable against a light exposure.
Admixing some kind of pharmaceutical additives with KMD-3213 results in incompatibility and yields degradation products. For example, compatibility between KMD-3213 and lactose, which is most popularly used as a filler, is bad, and use of lactose as a filler gives undesirable dissolution properties and unsatisfactory hardness of tablets. Moreover, KMD-3213 has a potent adhesive property, and in the case of preparing a tablet or capsule, use of a lubricant is inevitable. On the contrary, the addition of such lubricants causes the problem of delaying in dissolution time. Accordingly, it is extremely difficult to prepare practically usable solid oral dosage form pharmaceuticals comprising, as an active ingredient, KMD-3213, its prodrug, pharmaceutically acceptable salt or pharmaceutically acceptable solvate thereof by conventional formulation methods.

Regarding such problems, patent literatures 1 and 2 do not disclose or suggest any method to solve the problems.
Patent literature 2 discloses a process for preparing capsules comprising, as an active ingredient, tamuslosin hydrochloride or alfuzosin hydrochloride. However, the pharmaceutical compositions of such capsules are quite different from those of the present invention. Moreover, pharmaceutical compositions of the present invention can not be prepared by processes disclosed in patent literature 2. Accordingly, patent literature 2 does not teach or suggest the present invention at all.
Patent literature 1: Japanese unexamined publication H06-220015 (page12, column21)
Patent literature 2: Japanese unexamined publication 2001-288115 (page3, column 3-4)

### DISCLOSURE OF THE INVENTION

The present invention provides a practically usable solid oral dosage form pharmaceutical for treating dysuria without affecting blood pressure, which comprises, as an active ingredient, KMD-3213, its prodrug, pharmaceutically acceptable salt or pharmaceutically acceptable solvate thereof, wherein said pharmaceutical has a high precision for content uniformity, good stabilities and excellent dissolution properties.

In cases where pharmaceuticals are administered orally, bioavailability of active ingredients contained therein is quite important, and exerting a constant efficacy is also required. For that purpose, assuring uniformity, i.e. bioeqivalence among formulation batches is required. In pharmacopoeias, procedures for testing disintegrating or dissolution properties of solid formulations are defined for assuring a constant quality and bioequivalence of the formulations. Accordingly, pharmaceuticals are requested to meet specifications as defined based on such tests.

Recently, dissolution testing is considered as an important means for estimating efficacy or safety profiles of pharmaceuticals. Particularly in the case of hardly soluble drug substances, dissolution properties rather than disintegration properties are more crucial for estimating the quality of pharmaceuticals comprising such substances.

In the light of bioequivalence, dissolution tests are desirable to carry out under a variety of testing conditions. However, it is difficult to define a specification of the dissolution tests based on various conditions, and ordinarily the dissolution tests are carried out under a condition in which pharmaceuticals are most likely to be non-bioequivalent. As a test medium in a dissolution test, test media in the physiological range of pH, i.e. pH 1 to 7, or water are generally used, while differences in formulations are detected clearly by using a test medium in which active ingredients are slowly released from the formulations. Water is sensitive to a change of pH. On the contrary, water is a test medium which can evaluate subtle differences in formulations or manufacturing processes. Accordingly, in cases where water can be used as a test medium in a dissolution test, it is desirable to use water in view of efficacies in tests, economical efficacies and effects on the environment.

KMD-3213 has relatively a high solubility in an acidic medium and is hardly soluble in a neutral medium such as water. Consequently, water is the most suitable test medium for evaluating non-bioequivalence on conducting a dissolution test. In developing a solid oral dosage form formulation comprising KMD-3213 as an active ingredient, it is desirable to find a formulation having a good dissolution property in water. In a pharmaceutical of the present invention, T85% is preferably not more than 60 minutes in a dissolution test according to method 2 (paddle method) of Japanese pharmacopoeia in a condition using water as a test medium and a paddle speed of 50rpm, more preferably T85% is not more than 30 minutes, and most preferably T85% is not more than 15 minutes.

Solid oral dosage form pharmaceuticals are desired to show good dissolution properties in the stomach except for cases where the pharmaceutical are enteric coated formulations due to their unstable properties in acidic conditions. Since KMD-3213 is stable in acidic conditions, solid oral dosage form formulations comprising KMD-3213 as an active ingredient are desired to show good dissolution properties in the first fluid, which is corresponding to gastric juice, in a dissolution test. Accordingly, in solid oral dosage form formulations of the present invention, T85% is preferably not more than 60 minutes in a dissolution test using the first fluid as in cases where the dissolution test is carried out using water, more preferably T85% is not more than 30 minutes, and most preferably T85% is not more than 15 minutes.

Active ingredients contained in pharmaceuticals exhibit generally their biological activities in a minute quantity of dosage. Therefore, for exerting a constant efficacy, it is important to make the content of active ingredients at a constant level and minimize a decrease in the content of the active ingredients during storage. For that purposes, it is desired to show a high content uniformity among formulation batches and high stabilities during storage.

KMD-3213 contained as an active ingredient in a solid oral dosage form pharmaceutical of the present invention has potent adhesive and electrostatic properties. Particularly, in cases where formulations are prepared by a dry process, electrostatic charges are generated by physical irritations caused through processes such as pulverization, agitation, blending, granulation and the like, which in turn cause a decrease in fluidity of pulverized, blended or granulated materials, worsen handling properties, and decrease precision for content uniformity of an active ingredient.

In the case of tablets or capsules, lubricants are added at the steps of filling or tabletting in consideration of handling properties, precision for filling and the like. KMD-3213 contained as an active ingredient in a solid oral dosage form pharmaceutical of the present invention has potent adhesive properties, and use of lubricants is inevitable. On the contrary, the use of the lubricants causes delaying in a dissolution time.

Furthermore, KMD-3213 contained as an active ingredient in a solid oral dosage form pharmaceutical of the'present invention is relatively unstable against a light exposure, and requires a careful handling. In such cases, formulations are generally stored under a light-resistant packaging. However, opaque light-resistant packages are difficult to detect contaminations of foreign materials. Moreover, when patients are actually taking formulations wrapped with light-resistant packages, the formulations are occasionally stored with pulled out of light-resistant packages. Accordingly, formulations, which can be stored without a light-resistant packaging and are highly photostable, are desired.

The present inventors have eagerly investigated a solid dosage form pharmaceutical which comprises, as an active ingredient, KMD-3213, itsprodrug, pharmaceutically acceptable salt or pharmaceutically acceptable solvate thereof and are extremely useful for the treatment of dysuria, wherein said pharmaceutical has a high precision for content uniformity, excellent dissolution properties in water, or water and the first fluid and good stabilities.

As a result, the present inventors have found that use of lactose, which is most popularly used as a filler, causes the problems of delaying in a dissolution time, decreasing in the hardness of tablets and the like. Consequently, preferable formulations cannot be prepared by using lactose as a filler. On further investigation into fillers, the present inventors have found that use of D-mannitol as a filler provides an extremely preferable dissolution property.

Moreover, the present inventors have studied a variety of processes for preparing formulations, and have found out that formulations, which has satisfactory content uniformity without influenced by electrostatic charges and has good stabilities and excellent dissolution properties, are prepared through granulating by a wet process and regulating the amount of a lubricant and a mixing time. The present inventors have also found that in the cases of capsules, formulations with excellent dissolution profiles are prepared by admixing a lubricant in a specific ratio with another additive which is a solid with hydrophilic or surface-active properties. Furthermore, the present inventors have studied a photostable formulation to find out that the photo-degradations of KMD-3213 are well prevented by titanium oxide and photostable formulations can be prepared by using a capsule containing titanium oxide or a coating agent containing titanium oxide. Based on these findings, the present invention has been accomplished.

In many cases, compounds contained as an active ingredient are relatively unstable, and blending such compounds with pharmaceutical additives which are used for preparing solid dosage form formulations, often causes incompatibility such as discoloring, decomposing and the like. However, it is difficult to estimate compatibility between a pharmaceutical additive and an active ingredient beforehand.

The present inventors have firstly investigated compatibility between KMD-3213 contained as an active ingredient of the present pharmaceutical and various kind of pharmaceutical additives used in the preparation of solid dosage form formulations, and then selected pharmaceutical additives which does not cause discoloring or decomposing. Thereafter, the present inventors have studied whether or not the selected pharmaceutical additives can be combined with each other without causing incompatibility and are suitable formanufacturability.

As a result of studies on fillers, lactose most popularly used as a filler does not cause incompatibility but decreases in dissolution properties and the hardness of tablets. For that reasons, it is difficult to prepare a preferable formulation by using lactose as a filler. The delaying in a dissolution time caused by lactose is improved by adding crystalline cellulose while the hardness of tablets is not improved with the addition of crystalline cellulose. Moreover, crystalline cellulose causes incompatibility on blending with KMD-3213 and yields degradation products. Consequently, crystalline cellulose is not suitable for preparing a solid dosage form pharmaceutical of the present invention. On further investigation into fillers, the present inventors have found that D-mannitol is suitable for compatibility and manufacturability and provides an extremely good dissolution property, and accordingly is most suitable as a filler.

As for a disintegrant, calcium carboxymethylcellulose and carboxymethylcellulose are not suitable for causing a large degree of incompatibility while starch, low-substituted hydroxylpropylcellulose, partially pregelatinized starch or the like are preferred. Examples of starch include corn starch and the like. Examples of partiallypregeratinized starch include starch 1500 (registered mark, Japan Colorcon Co., Ltd.), PCS (registered mark, Asahi Chemical Industry Co., Ltd.) and the like.

As for a binder, hydroxypropylmethylcellulose and hydroxypropylcellulose are not suitable for causing a small degree of incompatibility.

As for a lubricant, magnesium stearate, calcium stearate and talc do not cause incompatibility and are preferred.

As for a surfactant, macrogol (polyethyleneglycol), polyoxyethylene(105)polyoxypropylene(5)glycol and triethyl citrate are not suitable for causing a large degree of incompatibility.

Based on these findings as described above, the preferred additives are selected. Then, processes for preparing formulations according to conventional procedures are investigated. Firstly, in cases where formulations are prepared by dry processes, pulverized, blended or granulated materials, which are prepared at pulverization, blending or granulation processes, generate electrostatic charges and decrease in fluidities of the materials. As a result, particularly in the case of preparing capsules, handling properties are worsened at the filling process, and uniformity of the fill volume and precision for filling are worsened.

For improving handling properties or precision for filling, lubricants are generally used at the filling process in capsules or at the tabletting process in tablets. KMD-3213 has inherently potent adhesive properties, and particularly in the case of dry processes, electrostatic charges are generated and fluidities of blended or granulated materials are worsened as described above, which result in the use of much more amount of lubricants. However, lubricants have generally water repellent properties and the use of lubricants causes delaying in a dissolution time.

The present inventors have intensively investigated the kind, combination or ratio of additives, manufacturing processes and the like, and have found highly practically usable formulations which have suitable handling properties for manufacturing processes, high precision for content uniformity and excellent dissolution properties and are useful for exerting biological activities of KMD-3213 effectively.

Firstly, the present inventors have found that delaying in a dissolution time is prevented to some extent by decreasing the amount of lubricants or shortening a mixing time. More specifically, good dissolution properties are accomplished by decreasing the amount of lubricants in not more than about 1%, more preferably in the range of about 0.6% to about 0.8%, and mixing shortly for a period of about 3 to about 5 minutes. Then, formulations with good fluidities of blended materials, satisfactory handling properties and high precision for filling can be prepared by granulating through a wet process in place of a dry process, using lubricants in an amount of not more than 1% and mixing for a period of about 3 minutes.

However, KMD-3213 contained as an active ingredient in a pharmaceutical of the present invention has potent adhesive properties, and in cases where capsules are prepared by using a lubricant in an amount of not more than about 1%, it is at high risk for causing a filling problem such as sticking. Regarding such problems, the present inventors have investigated a process for improving the delay in a dissolution time even in the case of using a lubricant in an amount of not less than 1%, and have found out that the delaying in a dissolution time can be prominently improved by blending a solid additive having hydrophilic or surface-active properties and thereby formulations with good dissolution properties can be prepared.

The effect of improving the delay in a dissolution time by the above mentioned additive differs depending on a combination of the additive with a lubricant. For example, where magnesium stearate is used as a lubricant, sodium lauryl sulfate is most preferred for the improving effect, and sucrose ester of fatty acid, light anhydrous silicic acid and polyoxyethylene(105)polyoxypropylene(5)glycol are unsatisfactory for the effect. For exerting a satisfactory improving effect, it is preferred to use in an amount of about 0.1 to about 2 parts, more preferably about 0.5 parts of sodium lauryl sulfate based on 1 part of magnesium stearate where dissolution properties can be maintained at a desirable level.

The effect of improving the delay in a dissolution time by sodium lauryl sulfate varies greatly depending on addition methods. For example, where sodium lauryl sulfate is dissolved in water and added together with bound water at a granulating process (hereinafter referred to as "addition during granulation", dissolution rates are decreased at a point immediately after starting a dissolution test (5 minutes value). On further investigation, the present inventors have found out that the delaying at an initial rise can be prevented by adding sodium lauryl sulfate together with a lubricant after a granulating process (hereinafter referred to as "addition after granulation".

KMD-3213 contained as an active ingredient in a solid oral dosage formpharmaceutical of the present invention is relatively unstable against a light exposure and the amount of the active ingredient is decreased with time depending on storage conditions. Accordingly, KMD-3213 requires a careful storage condition and handling. In such cases, formulations are generally stored under a light-resistant packaging, while opaque light-resistant packages are difficult to detect contaminations of foreign materials and are accordingly at high risk for overlooking defective product. Moreover, when patients are actually taking formulations wrapped with light-resistant packages, the formulations are occasionally stored with pulled out of light-resistant packages. Accordingly, formulations, which can be stored without a light-resistant packaging and are highly photostable, are desired.

The present inventors have investigated a preferable light-shielding material for blending in capsules or coating agents, and have found out that titanium oxide is most preferred as a light-shielding material. Highly photostable capsules or tablets can be prepared by using capsules containing titanium oxide or coating agents containing titanium oxide.

Photostabilities are evaluated as follows. Firstly, upper acceptance criteria for the amounts (%) of each photodegradation materials (hereinafter referred to as "related substance") and the total amounts (%) of all related substances are defined. Then, the photostabilities are evaluated by assessing whether or not the amounts of related substances are conformed to the acceptance criteria in the presence of standard light exposure. It is reported in JIS (Japanese Industrial Standards) that standard illumination levels are 300-750lux/hour in a hospital pharmacy where average lighting hours are about 8 hours/day and maximum shelf life of pharmaceuticals are 6 months. Accordingly, standard light exposure is estimated to be about 1.2 million lux/hour, which is calculated by considering a condition of 750 lux/hour as a maximum illumination level, about 8 hours as a daily lighting hour and 180 days as a light exposure period that is corresponding to an about 1.08 million lux/hour of light exposure, and its measurement deviation. In a guideline of ethical pharmaceuticals, photostability testing is required to carry out under an overall illumination of not less than about 1. 2 million lux/hour. Consequently, it is requested that ethical pharmaceuticals are stable under a light exposure of about 1.2 million lux/hour in a photostability test.

It is ascertained that there are at least 6 related substances in KMD-3213 contained as an active ingredient in a solid oral dosage form pharmaceutical of the present invention. A provisional specification is defined as not more than 4% for the largest quantity of related substance a, not more than 1% for each of related substances b to f and not more than 5% for total amounts of all related substances including minute quantities of other related substances. The present inventors have investigated a light-shielding capsule or coating agent for conforming to a light exposure of about 1.2 million lux/hour.

As a result, titanium oxide is most preferred as a light-shielding material, and highly photostable solid dosage form pharmaceuticals are prepared by using capsules containing titanium oxide or coating agents containing titanium oxide.

Light-shielding effects increase with blending amounts of titanium oxide while the strength of capsules decreases with blending amounts of titanium oxide. Preferred blending amounts are appropriately determined depending on the size of pharmaceuticals. For exerting preferable light-shielding effects in capsules, the blending amount of titanium oxide is not less than about 3%, more preferably about 3.4-3.6%. For tablets, the blending amount of titanium oxide is determined by the surface area of tablets, the amount of coating agents and the like. For exerting preferable light-shielding effects, the coating amount of titanium oxide is generally not less than 0.5mg/square cm, more preferably 1.1mg/square cm based on the surface area of tablets.

Regarding pharmaceutical compositions comprising, as an active ingredient, KMD-3213, pharmaceutically acceptable salt or pharmaceutically acceptable solvate thereof, there are only general descriptions in patent literatures 1 and 2 which do not teach or suggest any specific pharmaceutical composition.

As described above, there are many problems to solve for providing a practically usable solid oral dosage form pharmaceutical comprising, as an active ingredient, KMD-3213, its prodrug, pharmaceutically acceptable salt or pharmaceutically acceptable solvate thereof according to conventional formulation methods. Patent literatures 1 and 2 do not disclose or suggest the problems and any method to solve such problems.

KMD-3213 contained as an active ingredient in a solid oral dosage form pharmaceutical of the present invention is a known compound and can be prepared according to procedures as described in patent literature 1.

Examples of pharmaceutical acceptable salts of KMD-3213 contained as an active ingredient in a solid oral dosage form pharmaceutical of the present invention include acid addition salt formed with mineral acids such as hydrochloric acid, hydrobromic acid, hydroiodic acid, sulfuric acid, nitric acid, phosphoric acid and the like; acid addition salts formed with organic acids such as acetic acid, propionic acid, butyric acid, oxalic acid, citric acid, succinic acid, tartaric acid, fumaric acid, malic acid, lactic acid, adipic acid, benzoic acid, salicylic acid, methanesulfonic acid, benzenesulfonic acid, p-toluenesulfonic acid, glutamic acid, aspartic acid and the like. Examples of solvate include solvates with water, ethyl alcohol or the like.

Solid oral dosage form pharmaceuticals of the present invention such as capsules can be prepared as follows. KMD-3213, pharmaceutically acceptable salt or pharmaceutically acceptable solvate thereof is admixed with a filler, preferably D-mannitol, if required, an appropriate binder and disintegrator. Then, the mixture is kneaded with the addition of an aqueous solution of binder in an appropriate concentration, and if required, sieved to prepare a granule. Thereafter, a lubricant, preferably magnesium stearate and a solid additive with hydrophilic or surface-active properties, preferably sodium lauryl sulfate are added to the granule, in that case the lubricant being used in an amount of 0.5-2.0%, and the solid additive being used in a ratio of 1:10 to 20:10, more preferably 5:10 to 10:10, even more preferably 5:10 relativelytomagnesiumstearate. Then, mixing and filling into an appropriate capsule, preferably a capsule containing titanium oxide in a blending amount of not less than about 3%, more preferably about 3.4 to 3.6% provide capsules.

Tablets can be prepared as follows. A granule is prepared according to procedures analogous to those as described in capsules. Then, a lubricant, preferably magnesium stearate in an amount of not more than 1%, preferably about 0.6 to about 0.8%, more preferably about 0.7% is added to the granule. Then, mixing and tabletting by conventional methods provide uncoated tablets. Thereafter, the uncoated tablets are, if required, spray-coated with a coating solution which is prepared by dissolving or suspending a film-coating agent, a light-shielding material, preferably titanium oxide, a plasticizing material, if required, an appropriate lubricant, an agglomeration suppressing material and a coloring agent in a suitable solvent. It is sufficient that the amount of titanium oxide is not less than 0.5mg/square cm, more preferably 1.1mg/square cm based on the surface area of tablets.

KMD-3213 exhibits α₁-AR blocking activities with less affecting blood pressure and is extremely useful compound for the treatment of dysuria associated with prostate hypertrophy and the like. It is reported that prazosin hydrochloride and tamuslosin hydrochloride having α₁-AR blocking activities are also useful for the treatment of dusuria such as bladder celvix sclerosis,chronic prostatitis,neurogenic bladder and the like.

It has been expected that KMD-3213 is useful for the treatment of dysuria associated with urethra organized obstructions such as prostate hypertrophy, urethra stricture, urethra calculus, tumors and the like (hereinafter referred to as "prostate hypertrophy etc") and dysuria associated with disorders of urination control nerves as well as dysuria associated with urethra functional obstructions, which is not included in any dysuria described above, such as bladder celvix sclerosis, chronic prostatitis, unstable bladder and the like.

Dysuria associated with disorders of urination control nerves means dysuria caused by disorders of control nerves in the urethra or the bladder, for example, encephalopathy such as cerebrovascular disorders, brain tumors and the like, spinal cord disorders such as spinal cord injuries, peripheral nerve disorders such as diabetes mellitus, lumbar region spinal canal stenosis and the like. These disorders may occur in both men and women, and are generally called as neurogenic bladder.

Dysuria associated with urethra functional obstructions not accompanied with urethra organized disorders and disorders of urination control nerves means bladder celvix sclerosis, chronic prostatitis and unstable bladder as well as dysuria caused by urination difficulty, bladder cervix blockage, urethra syndrome, detrusor muscle-sphincter mascle cooperation insufficiency, chronic cystitis, prostatodynia, Hinman syndrome, Fowler syndrome, psychogenic dysuria, drug-induced dysuria, aging and the like. These disorders are generally called as lower urinary tract disorders.

The pharmaceuticals of the present invention have a high precision for content uniformity and excellent dissolution properties, and accordingly can exert the activities of KMD-3213 effectively. The pharmaceuticals of the present invention is extremely useful for the treatment of dysuria associated with urethra organized obstructions such as prostate hypertrophy etc; dysuria associated with disorders of urination control nerves such as neurogenic bladder; and dysuria associated with urethra functional obstructions such as lower tract disorders.

In the case of administering a pharmaceutical of the present invention, the dosage of an active ingredient is appropriately determined depending on the sex, age or body weight of the individual patient, the condition to be treated and the like, which is approximately in the range of 1 to 50mg, preferably 4 to 20mg per day per adult human.

The pharmaceutical of the present invention may further comprise, as an active ingredient, at least one selected from the group consisting of an α₁-adrenoceptor blocking agent, an anticholinergic agent, an antiinflammatory agent and an antibacterial agent other than KMD-3213 in addition with KMD-3213, pharmaceutically acceptable salt or pharmaceutically acceptable solvate thereof.

The pharmaceutical of the present invention may be used in combination with a pharmaceutical comprising, as an active ingredient, at least one selected from the group consisting of an α₁-adrenoceptor blocking agent, an anticholinergic agent, an antiinflammatory agent and an antibacterial agent other than KMD-3213.

In such cases, the dosage of pharmaceutically acceptable salt or pharmaceutically acceptable solvate thereof and the dosages'of an α₁-adrenoceptor blocking agent, an anticholinergic agent, an antiinflammatory agent and an antibacterial agent other than KMD-3213 may be suitably reduced.

### BRIEF DESCRIPTION OF THE DRAWINGS

Fig. 1 is a drawing which shows a relation between mixing time of magnesium stearate and delaying actions of magnesium stearate on a dissolution time wherein -•- is formulation A, -o- is formulation B with a mixing time of 1 min. (formulation B/1 min.), -□- is formulation B with a mixing time of 3 min. (formulation B/3 min.) and -◇- is formulation B with a mixing time of 7 min. (formulation B/7 min.). The ordinate shows dissolution rates (%) and the abscissa shows time in minutes.
Fig. 2 is a'drawing which shows the effects of various kinds of additives on delaying in a dissolution time caused by magnesium stearate wherein -•- is formulation A, -□- is formulation B,-o- is formulation C, -■- is formulation D, -◇-is formulation E, -Δ- is formulation F and -◊- is formulation G. The ordinate shows dissolution rates (%) and the abscissa shows time in minutes.
Fig. 3 is a drawing which shows a relation between mixing ratios of magnesium stearate to sodium lauryl sulfate and dissolution properties wherein -•- is formulation H, -□- is formulation I, -A- is formulation J, -ο- is formulation K and -◆- is formulation L. The ordinate shows dissolution rates (%) and the abscissa shows time in minutes.
Fig. 4 is a drawing which shows dissolution properties of formulations of examples 1 to 3 wherein -ο- is the formulation of example 1, -•- is the formulation of example 2 and -Δ- is the formulation of example 3. The ordinate shows dissolution rates (%) and the abscissa shows time in minutes.
Fig. 5 is a drawing which shows a relation between blending amounts of titanium oxide and photostabilities in' capsules containing titanium oxide wherein -•- is a control (stored in a light-shielding vessel), -Δ- is capsule A (containing 1.2% of titanium oxide), -■- is capsule B (containing 2.4% of titanium oxide) and -o- is capsule C (containing 3.6% of titanium oxide). The ordinate shows total amounts of all related substances (%) and the abscissa shows the quantities of light exposure (1000 lux/hour).

### BEST MODE FOR CARRYING OUT THE INVENTION'

The following examples and test examples illustrate the invention in further detail.

### Test Example 1

### Compatibility Test

KMD-3213 and a variety of pharmaceutical additives which are used for formulating oral solid dosage forms, were mixed and evaluated for compatibility with KMD-3213. The additives, which are used in a large amount such as a filler, disintegrant and binder, were mixed with KMD-3213 in the ratio of 1:1, and other additives, which are used in a small amount, were mixed in the ratio of 10:1. The mixtures were stored under the following conditions 1 and 2, and the changes on blending, i.e. incompability, were checked. Degradation products were detected by HPLC analysis according to the following HPLC conditions, and appearances were checked by visual examination.

### Storage Conditions

Condition 1: 40°C, 80% relative humidity and 3 weeks Condition 2: 40 °C, 75% relative humidity and 4 months Analytical Method

A mixture of KMD-3213 and a pharmaceutical additive, which is equivalent to about 5 mg of KMD-3213, was weighed accurately, and'the mixture was dissolved in methanol to make exactly a 10 mL of solution after 10 minutes sonication. 4 mL of the solution was pipetted, and methanol was added to make exactly a 5 mL of solution. The resulting solution was filtered through a membrane filter with a pore size of not more than 0.45 µm. This solution was used as a test solution.

5 µL of each test solutions were analyzed according to the following HPLC conditions. The ratio of the peak area of each related substances relatively to the peak area of the solutions excluding the peak area of solvent was calculated by an area percentage method.
HPLC Conditions:
Wavelength: 225 nm
Column: Capcell Pack C18 UG120 (Shiseido Co., Ltd.)
Column temperature: About 25 °C

Mobile phase: 6.8 g of potassium dihydrogen phosphate and 17.9 g of disodium hydrogen phosphate 12 hydrate were dissolved in water to make a 1000 mL of solution, then the solution was mixed with acetonitrile in the ratio of 7:3 to prepare a mobile phase Flow rate: 1.0 mL/min
Time span of measurement: 40 min

Tables 1 and 2 show'the results tested under the conditions 1 and 2 respectively.

As shown in tables 1 and 2, D-mannitol was most suitable as a filler, but microcrystalline cellulose was incompatible. As for disintegrants, corn starch was most suitable, and calcium carboxymethylcellulose and carboxymethylcellulose were incompatible remarkably. As for binders, hydroxypropylmethylcellulose and hydroxypropylcellulose were rather incompatible. As for surfactants, macrogol, Polyoxyethylene(105)polyoxypropylene (5)glycol and triethyl citrate were incompatible remarkably.

**Table 1 Condition 1: 40°C/80%RH, 3weeks**

| pharmaceutical additives | function | color change | degradation products (%) |
|---|---|---|---|
| D-Mannitol | filler | - | +0.44 |
| Lactose | ↓ | - | +0.54 |
| Microcrystalline cellulose | ↓ | - | +1.01 |
| Corn Starch | disintegrant | - | +0.23 |
| Low substituted Hydroxypropylcellulose | ↓ | - | +0.55 |
| Calcium Carboxymethyl-cellulose | ↓ | +++ | +3.57 |
| Carboxymethylcellulose | ↓ | +++ | +8.24 |
| Hydroxypropylmethylcellulose | binder | - | +0.83 |
| Hydroxypropylcellulose | ↓ | + | +0.76 |
| Magnesium stearate | lubricant | - | +0.92 |
| Calcium stearate | ↓ | - | +0.61 |
| Talc | ↓ | - | +0.38 |
| Macrogol (polyethyleneglycol) | surfactant | + | +1.55 |
| Polyoxyethylene(105) polyoxypropylene(5)glycol | ↓ | + | +0.73 |
| Triethyl Citrate | plasticizer | ++ | +2.37 |

**Table 2 Condition 2: 40 °C/75%RH, 4months**

| pharmaceutical additives | function | color change | degradation products (%) |
|---|---|---|---|
| D-Mannitol | filler | - | +0.25 |
| Lactose | ↓ | - | +0.47 |
| Microcrystalline cellulose | ↓ | - | +0.55 |
| Corn Starch | disintegrant | - | +0.18 |
| Low substituted Hydroxypropylcellulose | ↓ | - | +0.50 |
| Calcium Carboxymethyl-cellulose | ↓ | ++ | +2.31 |
| Carboxymethylcellulose | ↓ | +++ | +3.31 |
| Hydroxypropylmethylcellulose | binder | - | +0.79 |
| Hydroxypropylcellulose | ↓ | - | +0.44 |
| Magnesium stearate | lubricant | - | +0.32 |
| Calcium stearate | ↓ | - | +0.36 |
| Talc | ↓ | - | +0.27 |
| Macrogol | surfactant | - | +0.51 |
| Polyoxyethylene(105) polyoxypropylene(5)glycol | ↓ | - | +0.32 |
| Triethyl Citrate | plasticizer | - | +0.79 |

### Test Example 2

### Study of relationship between mixing time of magnesium stearate and delay in dissolution time

The correlation between mixing time and delaying in dissolution time was investigated by using capsules containing D-mannitolas a filler,partially pregelatinized starch (Starch 1500 (registered mark), Japan Colorcon Co., Ltd.) as a disintegrant and about 1.0% of magnesium stearate as a lubricant.

Each of capsules was prepared according to the formulations as showed in table 3, and their dissolution times were evaluated.

### Dissolution Test Method

The dissolution test was carried out using 1 capsule at a paddle speed of 50 revolutions per minute (rpm) according to Method 2 of Dissolution Test (Japanese Pharmacopeia), using a sinker and 500 mL of water as a test medium. 5 mL of the dissolved solution was taken at 5, 10, 15, 20 and 30 minutes after starting the test, and the same volume of test medium was filled immediately. The solutions taken at each point of time were filtered through a membrane filter with a pore size of not more than 0.45 µm. The first 4 mL of the filtrates was discarded, and the subsequent filtrate was used as a test solution.

Separately, about 0.01 g of KMD-3213was weighed accurately, and dissolved in water to make exactly a 100 mL of solution. 8 mL of the solution was pipetted, and water was added thereto to make exactly a 100 mL of solution which was used as a standard solution.

The test was carried out using 100 µL of each test solutions and the standard solution according to the following Liquid Chromatography conditions. Dissolution rates were calculated from the peak area of KMD-3213 in the test solutions and the standard solution. In addition, the dissolution rates were calculated as the mean average of 6 samples for each of capsules. HPLC Conditions:
Wavelength: 270 nm
Column: Inertsil ODS-3 (GL Sciences Co., Ltd.)
Column temperature: About 25 °C
Mobile phase: 3.9 g of sodium dihydrogen phosphate dihydrate and 2.5 mL of an aqueous solution of phosphoric acid (1 in 20) were dissolved in water to make a 1000 mL of solution, then the solution was mixed with acetonitrile in the ratio of 5:2 to prepared a mobile phase.

### Flow rate: 1.0 mL/min

- In the cases of preparing capsules of formulation B containing magnesium stearate, capsules were prepared by pulling out the mixture at a time of 1, 3, 5, and 7 minutes after starting mixing, and filling each of the mixtures into a capsule shell by hand.

As shown in Figure 1, the delaying in dissolution time of formulation B (mixing time: 1 minute) was observed slightly. As for formulation B (mixing time: 3 minutes), the dissolution time was delayed remarkably.

**Table 3**

| components | formulation A | formulation B |
|---|---|---|
| KMD-3213 | 4.0 | 4.0 |
| D-Mannitol | 169.2 | 169.2 |
| Partially pregelatinized starch (Starch 1500) | 10.0 | 10.0 |
| Magnesium stearate | | 1.8 |
| Total weight | 183.2 | 185.0 |

### Test Example 3

### Study of improving effects of pharmaceutical additives on the delay in dissolution time caused by magnesium stearate.

Improving effects of a variety of additives on delaying in dissolution time caused by the addition of 1% magnesium stearate was investigated for capsules. Capsules were prepared by adding the same amount of testing additives as magnesium stearate to formulation B in test example 2. The dissolution time of the capsules were measured according to the same test method as described in test example 2.

For preparing capsules, granules were firstly prepared, and then the additives, together with magnesium stearate, were added to the granules and mixed for 5 minutes.

As shown in Figure 2, only sodium lauryl sulfate (Formulation C) improved the delay in dissolution time, and Formulation C showed immediate dissolution as in the case of Formulation A in which magnesium stearate is not used.

**Table 4**

| formulation | A | B | C | D | E | F | G |
|---|---|---|---|---|---|---|---|
| KMD-3213 | 4.0 | 4.0 | 4.0 | 4.0 | 4.0 | 4.0 | 4.0 |
| D-Mannitol | 169.2 | 169.2 | 169.2 | 169.2 | 169.2 | 169.2 | 169.2 |
| Partially pre-gelatinized starch (Starch 1500) | 10.0 | 10.0 | 10.0 | 10.0 | 10.0 | 10.0 | 10.0 |
| Magnesium stearate | | 1.8 | 1.8 | 1.8 | 1.8 | 1.8 | 1.8 |
| Sodium Lauryl Sulfate | | | 1.8 | | | | |
| Sucrose Ester of Fatty Acid (Stearic Acid) | | | | 1.8 | | | |
| Sucrose Ester of Fatty Acid (Palmitic Acid) | | | | | 1.8 | | |
| Light Anhydrous Silicic Acid | | | | | | 1.8 | |
| Polyoxyethylene(105) polyoxypropylene(5) glycol | | | | | | | 1.8 |
| total weight | 183.2 | 185.0 | 186.8 | 186.8 | 186.8 | 186.8 | 186.8 |

### Test Example 4

### Study on influence of the ratio of magnesium stearate and sodium lauryl sulfate on the dissolution time of capsules

Correlation between the ratio of magnesium stearate and sodium lauryl sulfate, which showed good improving effect on delaying in dissolution time caused by the addition of magnesium stearate, and dissolution properties of capsules was investigated. Capsules were prepared according to the formulations as shown in Table 5, and their dissolution times were evaluated according to method 2 (paddle method) of Japanese Pharmacopoeia in a condition using water as a test medium, which was described in the following test method. HPLC conditions were the same as those in Test Example 2.

### Dissolution Test Method

Dissolution test was carried out using 1 capsule at a paddle speed of 50 revolutions per minute (rpm) according to Method 2 of Dissolution Test (Japanese Pharmacopeia), using a sinker and 500 mL water as a test medium. 5 mL of the dissolved solution was taken at 5, 10, 15, 20, and 30 minutes after starting the test, and the same volume of test medium was filled immediately. After the solutions taken at each point of time were centrifuged at 3000 revolutions per minute for more than 5 minutes, 10 µL of concentrated hydrochloric acid was added to the supernatant of the centrifuged solutions, and the resulting solution was used as a test solution.

Separately, about 0. 01 g of KMD-3213 was weighed accurately and dissolved in 0.1 N hydrochloric acid to make exactly a 100 mL of solution. 2 mL of the solution was pipetted, and 0.1 N hydrochloric acid was added to make exactly a 100 mL of solution which was used as a standard solution.

For preparing capsules, granules were firstly prepared, and then the additives, together with magnesium stearate, were added to the granules and mixed for 5 minutes.

The dissolution rates were calculated as the mean average of 6 samples for each of capsules.

As shown in Figure 3, formulation I containing 10% sodium lauryl sulfate based on magnesium stearate showed good improving effect on dissolution property, and almost improved delaying in dissolution'time.

**Table 5**

| formulation | H | I | J | K | L |
|---|---|---|---|---|---|
| the ratio of Magnesium stearate to Sodium Lauryl Sulfate | 10:0 | 10:1 | 10:3 | 10:5 | 10:10 |
| KMD-3213 | 2.0 | 2.0 | 2.0 | 2.0 | 2.0 |
| D-Mannitol | 134.4 | 134.4 | 134.4 | 134.4 | 134.4 |
| Partially pregelatinized starch (PCS) | 26.0 | 26.0 | 26.0 | 26.0 | 26.0 |
| Partially pregelatinized starch (Starch 1500) | 9.0 | 9.0 | 9.0 | 9.0 | 9.0 |
| Magnesium stearate | 1.8 | 1.8 | 1.8 | 1.8 | 1.8 |
| Sodium Lauryl Sulfate | | 0.18 | 0.54 | 0.9 | 1.8 |
| total weight | 173.2 | 173.38 | 173.74 | 174.1 | 175.0 |

### Example 1

### Capsule containing 2.0mg of KMD-3213

2.0 parts of KMD-3213, 134.4 parts of D-mannitol, 26.0 parts of partially pregelatinized starch (PCS (registered mark), Asahi Chemical Industry Co., Ltd.) and 9.0 parts of partially pregelatinized starch (Starch 1500 (registered mark), Japan Colorcon Co., Ltd.) were mixed sufficiently. Appropriate amount of water was added thereto and the mixture was granulated. The granule was dried using a fluid bed dryer at an inlet air temperature of 60°C until the exhaust air reaches 40 °C, and sieved. A mixture of 1.8 parts of magnesium stearate and 1.8 parts of sodium lauryl sulfate was added to the sieved granules and mixed for 5 minutes, and the mixture was filled into a capsule shell to prepare a capsule containing 2.0mg of KMD-3213.

### Example 2

### Capsule containing 4mg of KMD-3213

4.0 parts of KMD-3213, 132.4 parts of D-mannitol, 26.0 parts of partially pregelatinized starch (PCS (registeredmark), Asahi Chemical Industry Co., Ltd.) and 9.0 parts of partially pregelatinized starch (Starch 1500 (registered mark), Japan Colorcon Co., Ltd.) were mixed sufficiently. Appropriate amount of water was added thereto and the mixture was granulated. The granule was dried using a fluid bed dryer at an inlet air temperature of 60 °C until the exhaust air reaches 40 °C, and sieved. A mixture of 1.8 parts of magnesium stearate and 1.8 parts of sodium lauryl sulfate were added to the sieved granules and mixed for 5 minutes, and the mixture was filled into a capsule shell to prepare a capsule containing 4mg of KMD-3213.

### Example 3

### Tablet containing 4.0mg of KMD-3213

4.0 parts of KMD-3213, 117.0 parts of D-mannitol, 7.0 parts of low substituted hydroxypropylcellulose (L-HPC (registered mark), Shin-Etsu chemical Co., Ltd.) were mixed sufficiently. A 12% aqueous solution of hydroxypropylcellulose (4 parts of hydroxypropylcellulose and about 30 parts of water) was added thereto and the mixture was granulated. The granule was dried using a fluid bed dryer at an inlet air temperature of 60°C until the exhaust air reaches 40 °C, and dry-sized and sieved. 1. 0 part of magnesium stearate was added to the granule and mixed for 3 minutes. The mixture was tabletted and coated with a coating agent to prepare a tablet containing 4.0mg of KMD-3213.

### Test Example 5

### Study on dissolution time

For the capsules or tablet as described in Examples 1 to 3, dissolution test was carried out according to the following dissolution test method. HPLC conditions was the same as those in test example 2.

### Dissolution Test Method

The test was carried out using 1 tablet or 1 capsule put into a sinker at a paddle speed of 50 revolutions per minute according to Method 2 of Dissolution Test (Japanese Pharmacopeia), using a 500 mL of water as a test medium. 5 mL of the dissolved solution was taken at 5, 10, 15, 20, and 30 minutes after starting the test, and the same volume of test medium was filled immediately. After the solutions taken at each point of time were centrifuged at 3000 revolutions per minute for more than 5 minutes. 10 µL of concentrated hydrochloric acid was added to the supernatant of the centrifuged solution, and the subsequent solution was used as a test solution.

Separately, about 0.01 g of KMD-3213 was weighed accurately, and dissolved in 0.1 N hydrochloric acid to make exactly a 100 mL of solution. In the case of dosage forms containing 2mg of KMD-3213 in example 1, 2 mL of the solution was pipetted, and 0.1 N hydrochloric acid was added to make exactly a 100 mL of solution which was used as a standard solution. In the case of dosage forms containing 4.0mg of KMD-3213 in examples 2 and 3, 4 mL of the solution was pipetted, and 0.1 N hydrochloric acid was added to make exactly a 100 mL of solution which was used as a standard solution.

The test was carried out using 100 µL of each test solutions and the standard solution according to the following Liquid Chromatography conditions. Dissolution rates were calculated from the peak area of KMD-3213 in the test solutions and the standard solution. In addition, the dissolution rates were calculated as the mean average of 6 samples for each capsule or tablet.

HPLC Conditions:
Wavelength: 270 nm
Column: Inertsil ODS-3 (GL Sciences Co., Ltd.)
Column temperature: About 25°C
Mobile phase: 3.9 g of sodium dihydrogen phosphate dihydrate and 2.5 mL of an aqueous solution of phosphoric acid (1 in 20) were dissolved in water to make a 1000 mL of solution, then the solution was mixed with acetonitrile in the ratio of 5 : 2 to prepare a mobile phase.

### Flow rate: 1.0 mL/min

As shown in Figure 4, all of the dosage forms of examples 1-3 showed not less than 90% dissolution rate after starting test, and their 85% dissolution times were not more than 10 minutes.

### Test Example 6

### Photostability test of capsule containing titanium dioxide.

Photostability test was carried out for capsules which were prepared according to the procedures as described in example 1 using capsule shells containing 1.2% (CapsuleA), 2.4% (Capsule B) and 3. 6% (Capsule C) of titanium dioxide. Inaddition, a capsule, prepared using a capsule shell containing 1.2% of titanium oxide, was packed in a blister package and aluminum pouch for shading, and the capsule was also tested as a blind control.

The contents filled in the capsules were taken out at the beginning of the test and after light exposures of 0.672 and 1.2 million lux/hour overallillumination, and their appearances and the amounts of photo-degradation products (related substances) were evaluated. The amounts of photo-degradation products were determined according to the following HPLC conditions, and the changes of color were observed by visual examination.

### Assay of photo-degradation products

The contents of 5 testing capsules were taken out and put into a 50 mL of measuring flask. The empty capsules were washed twice with a mobile phase, and the washed solutions were put into the flask. About 30 mL of mobile phase was added to the flask and the mixture was shaked for 15 minutes. Thereafter, a mobile phase was added thereto to make exactly a 50mL of solution, and the solution was filtered through a membrane filter with a pore size of not more than 0.45 µm. The first 2 to 3 mL of the filtrate was discarded and the subsequent filtrate was used as a test solution. 25 µL of each test solutions were used for the following HPLC analysis. The peak area of the solutions was determined by an automatic integration method, and the ratio of the peak area of each related substances relatively to the peak area of KMD-3213 was calculated by an area percentage method. HPLC Conditions:
Wavelength: 225 nm
Column: Inertsil ODS-3 (GL Sciences Co., Ltd.)
Column temperature: About 25 °C
Mobile phase: 3.9 g of sodium dihydrogen phosphate dihydrate and 2.5 mL of an aqueous solution of phosphoric acid (1 in 20) were dissolved in water to make a 1000 mL of solution, and the solution was mixed with acetonitrile in the ratio of 5: 2 to prepare a mobile phase.

### Flow speed: Adjust retention time of KMD-3213 to 7 minutes Time span of measurement: 30 min

As shown in Figure 5 and Table 6, capsule A containing 1.2% of titanium dioxide was not conformed to the specification regarding appearance and the total amounts of all related substances after a light exposure of about 0. 672 million lux/hour overall illumination. Capsule B containing 2.4% of titanium dioxide was not also conformed to the specification after a light exposure of about 1.2 million lux/hour overall illumination. On the contrary, capsule C containing 3.6% of titanium dioxide was most stable and conformed to the specification regarding appearance and the total amounts of all related substance.

**Table 6**

| **sample** | **illumi-nation (million lux/hr)** | **Amount of related substance (%)** | | | | | | | | **appearance** |
|---|---|---|---|---|---|---|---|---|---|---|
| | | **a** | **b** | **c** | **d** | **e** | **f** | **others** | **total** | |
| **Capsule A** | 0 | 0.13 | | | 0.04 | | 0.04 | 0.07 | 0.28 | white |
| | 0.672 | 2.28 | 0.31 | 0.31 | 0.50 | 0.99 | 0.04 | 0.42 | 4.85 | yellowish white |
| | 1.248 | 3.52 | 0.49 | 0.52 | 0.68 | 1.61 | 0.04 | 0.68 | 7.54 | pale yellow |
| **Capsule B** | 0 | 0.15 | | | 0.02 | | 0.04 | 0.07 | 0.28 | white |
| | 0.672 | 1.55 | 0.19 | 0.21 | 0.40 | 0.69 | 0.04 | 0.30 | 3.38 | white |
| | 1.248 | 2.38 | 0.33 | 0.35 | 0.54 | 1.10 | 0.04 | 0.40 | 5.14 | yellowish white |
| **Capsule C** | 0 | 0.15 | | | 0.02 | | 0.04 | 0.07 | 0.28 | white |
| | 0.672 | 1.29 | 0.16 | 0.16 | 0.35 | 0.54 | 0.04 | 0.23 | 2.77 | white |
| | 1.248 | 1.93 | 0.26 | 0.27 | 0.47 | 0.87 | 0.04 | 0.31 | 4.15 | white |
| **Control** | 0 | 0.13 | | | 0.04 | | 0.04 | 0.07 | 0.28 | white |
| | 0.672 | 0.21 | | | 0.02 | | 0.04 | 0.04 | 0.31 | white |
| | 1.248 | 0.16 | | | 0.02 | | 0.04 | 0.04 | 0.26 | white |

### INDUSTRIAL APPLICABILITY

Solid oral dosage form pharmaceuticals of the present invention have suitable handling properties for manufacturing processes, good content uniformity and excellent dissolution properties, and are highly practically usable as a solid oral dosage form pharmaceutical for the treatment of dysuria. Solid oral dosage form pharmaceuticals of the present invention have good handling properties at the filling process for capsules or at the tabletting process for tablets, high precision for the content of an active ingredient and stabilities. Moreover, solid oral dosage form pharmaceuticals of the present invention have constant and excellent dissolution properties in a dissolution test using water in which the active ingredient is most hardly soluble and the pharmaceuticals are most likely to be non-bioequivalent. Accordingly, solid oral dosage form pharmaceuticals of the present invention are extremely useful as a solid oral dosage form pharmaceutical for the treatment of dysuria.

## Claims

1. A tablet for the treatment of dysuria, which is coated with a light-shielding coating agent containing titanium oxide and comprises a granule containing a) as an active ingredient, an indoline compound having an α₁-adrenoceptor blocking activity and represented by the formula: 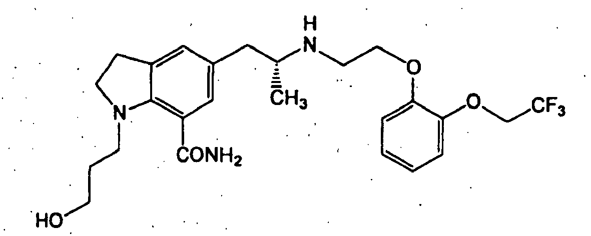
b) D-mannitol as a filler, c) low substituted hydroxypropylcellulose as a disintegrator, and
d) hydroxypropylcellulose as a binder,
wherein 85% dissolution time is not more than 15 minutes in a dissolution test according to method 2 (paddle method) of the Japanese pharmacopoeia in a condition using water as a test medium and a paddle speed of 50rpm.

2. The tablet according to claim 1, wherein the tablet is coated with not less than 0.5 mg/square cm of titanium oxide as a light-shielding coating agent based on the surface area of a tablet.

3. The tablet according to claim 1, wherein the tablet is coated with not less than 1.1 mg/square cm of titanium oxide as a light-shielding coating agent based on the surface area of tablet.

4. The tablet according to claim 1, which further comprises a lubricant.

5. The tablet according to claim 4, wherein the lubricant is magnesium stearate, calcium stearate or talc.

6. The tablet according to claim 5, wherein the lubricant is magnesium stearate.

7. A method for preparing a tablet, comprising the steps of (1) granulating a mixture of a) a compound represented by the formula: 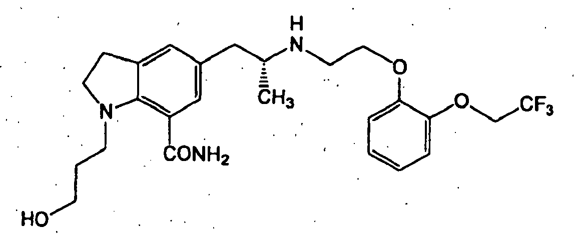
b) D-mannitol and c) low substituted hydroxypropylcellulose with a binder solution of hydroxypropylcellulose by a wet-granulation process;
(2) mixing the granular material obtained in step (1), and a lubricant selected from magnesium stearate, calcium stearate or talc, followed by tabletting; and
(3) coating the uncoated tablet obtained in step (2) with not less than 0.5 mg/square cm of titanium oxide as a light-shielding coating agent based on the surface area of tablet.

8. The method according to claim 7, comprising the step of (3) coating the uncoated tablet obtained in step (2) with not less than 1.1 mg/square cm of titanium oxide as a light-shielding coating agent based on the surface area of tablet.
